# EUROPEAN PATENT APPLICATION

(11) **EP 2 399 901 A1**
(43) Date of publication of application: **28.12.2011**
(21) Application number: 10006507.7
(22) Date of filing: 23.06.2010
(51) Int. Cl.: C07C 237/22, C07C 271/22

(54) **Intermediate for producing lacosamide and a process for its preparation and conversion to lacosamide**

(71) Applicant: Archimica GmbH, 65929 Frankfurt am Main (DE)
(72) Inventor: Wisdom, Richard, 65817 Eppstein (DE); Jung, Jörg, 65439 Flörsheim (DE); Meudt, Andreas, 65205 Wiesbaden (DE)
(74) Representative: Schweitzer, Klaus

(57) **Abstract**

The invention relates to R)-1-Benzylcarbamoyl-2-hydroxy-ethyl)-carbamic acid tert-butyl ester (compound III) with an ee of greater than 90%. 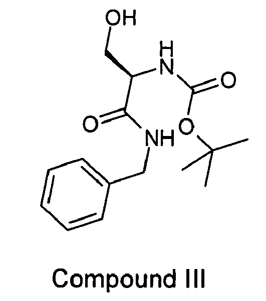 and a process for producing the same

## Description

(R)-2-Acetamido-N-benzyl-3-methoxypropionamide, also known as lacosamide, is effective in the treatment of pain, osteoarthritis, migraine and epilepsy. Lacosamide has the structure given below (I).

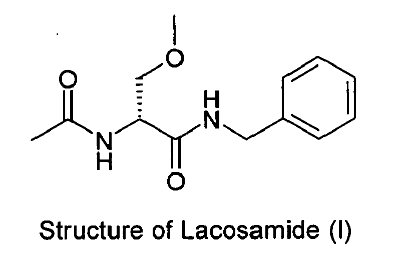

The product is described and claimed in W09733861. This document describes different synthetic methods. Starting with (D)-serine, this amino acid is converted to (R)-N-benzyl-2-acetamidohydracrylamide and then methylated using methyl iodide and silver oxide to produce the required product (Scheme 1).

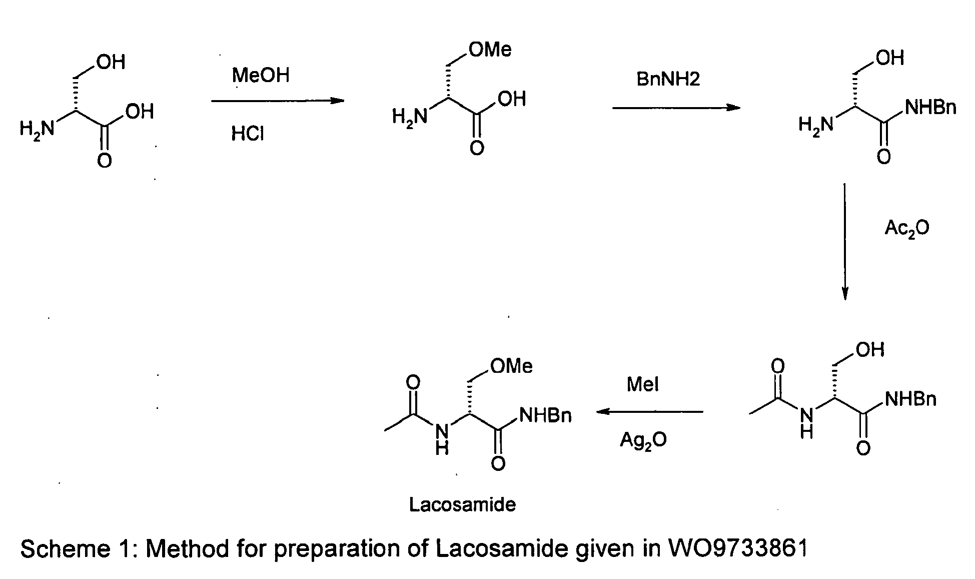

Alternatively; D-serine is first acetylated, the resulting N-acetyl amino acid coupled with benzylamine (BnNH2) under conditions described in JACS 1967, 89, 5012-7 via a mixed anhydride and finally methylated with a combination of methyl iodide and silver oxide (Scheme 2).

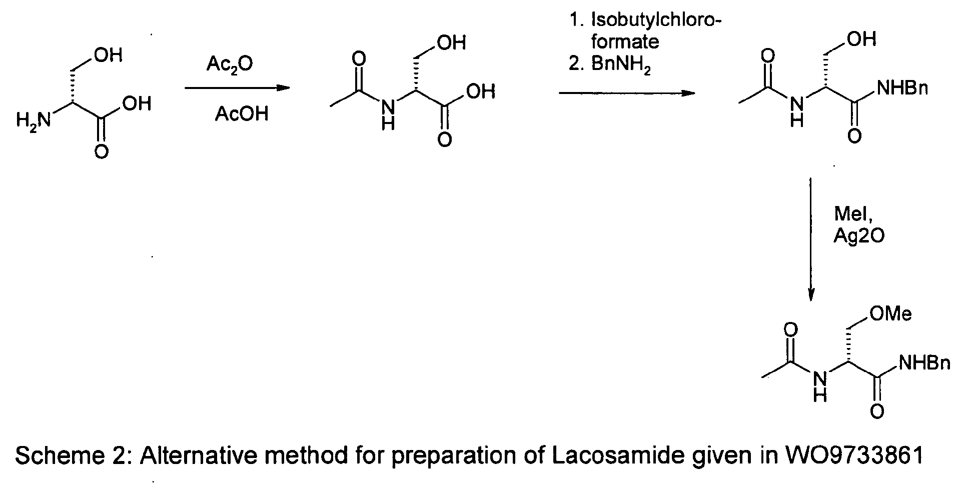

In a further alternative approach to prepare lacosamide described in WO9733861 , the amino group of D-serine is first protected with a carboxbenzoxy- group (Cbz), followed by 'O'-methylation and subsequent reactions to the desired product, eg benzylamination at the carboxylic acid function, de-protection, and acetylation of the amine function (Scheme 3). This route is also described in US6048899; which additionally provides a similar approach with inversion of steps 2 and 3, i. e. the amide formation is performed prior to the methylation.

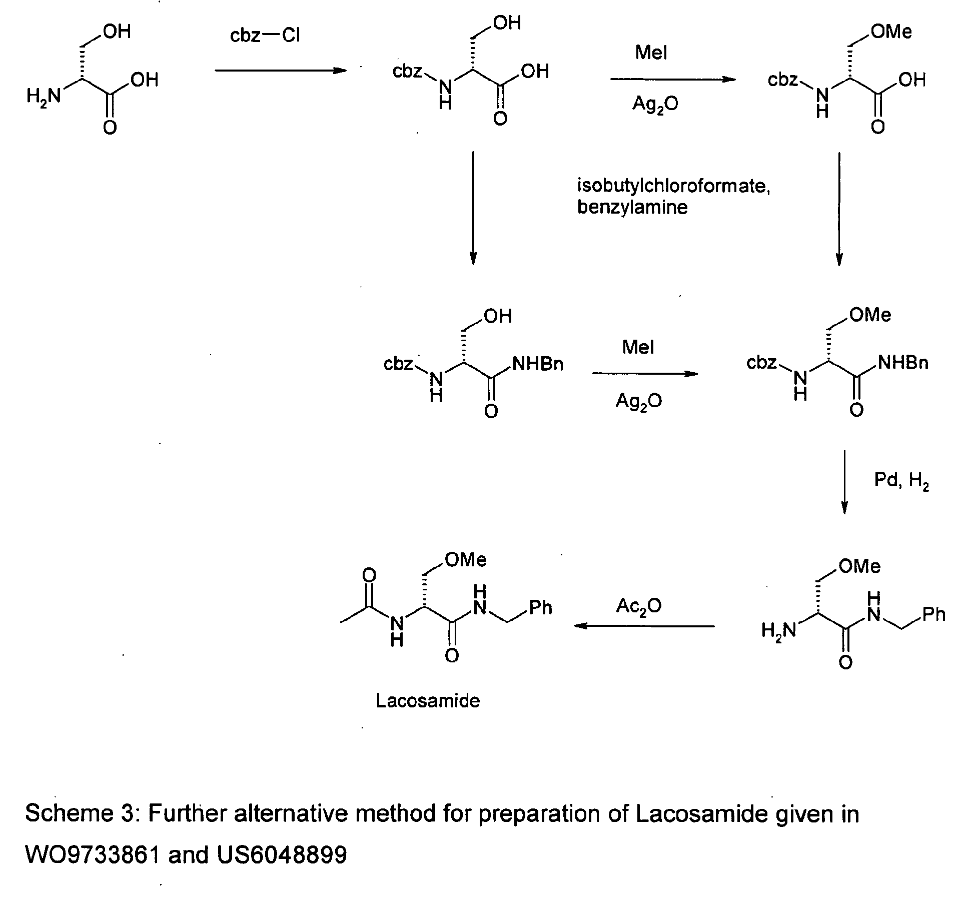

However, all those processes suffer from use of large quantities of silver, which is both expensive and must be removed from the final product. Additionally partial racemisation of the expensive chiral centre is observed in several cases, which lowers yields.

A further process for the production of lacosamide has also been described in EP2067765 A1. This uses the very bulky trityl- protecting group for protection of the serine, thereby minimising the potential for racemisation at the chiral centre during the subsequent methylation reaction (Scheme 4).

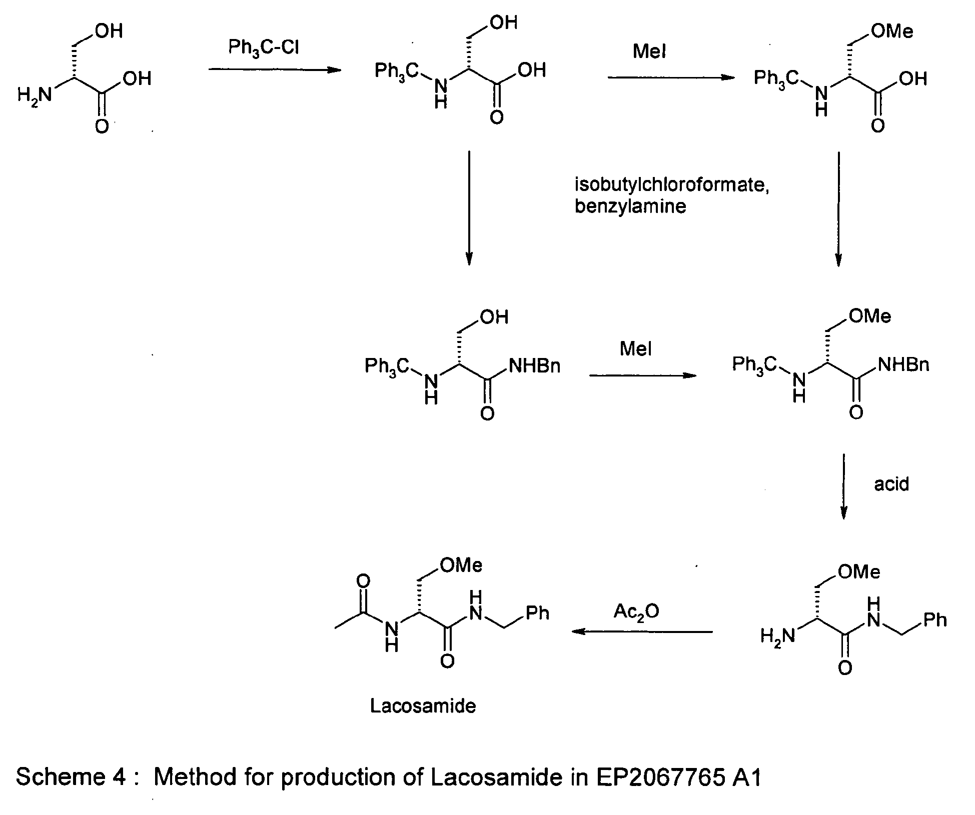

Although the use of expensive silver oxide is not longer necessary using trityl as protecting group, the necessity to perform many transformations on "tritylated" materials makes the process less effective from an economic point of view.

Alternatively WO 2006/037574 also describes a process for lacosamide production starting from D-serine. In this process, the amine group is protected with a boc (tert-butoxy-carbonyl-) group and the methylation of the amino acid is carried out either using a phase transfer catalyst or a methylation agent in combination with an organolithium reagent. After methylation, the boc protected (D)-methoxy-serine is worked through to the lacosamide via benzylamination, de-protection, and acetylation.

Recently WO2010052011 has been published which describes routes to racemic lacosamide (a mixture of (R)- plus (S)-2-acetamido-N-benzyl-3-methoxypropionamide). The racemic mixture is then separated using simulated bed chromatography (SMB) with a chiral phase, and the unwanted enantiomer is then racemised and recycled.

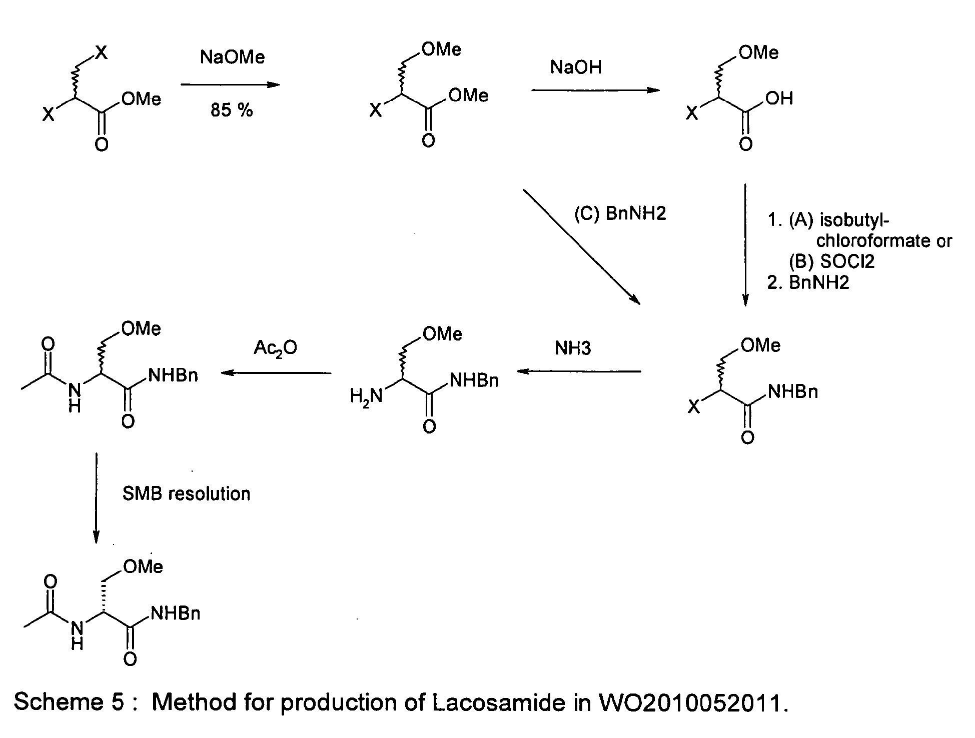

Despite these various approaches, there still remains a need for improved processes for the production of this important drug.

### Description of the Invention.

Surprisingly it has be found that boc-(D)-serine (II), which can readily be obtained through the reaction of di-tert-butyl dicarbonate with (D)-serine can be coupled readily with benzylamine without racemisation. The boc protected (R)-2-amino-N-benzyl-3-hydroxy-propionamide (III) thus formed may be cleanly methylated using standard methylation reagents such as dimethylsulfate to give (R)-boc-2-amino-N-benzyl-3-methoxy-propionamide (IV). In this novel route, formation of the benzylamide is carried out before methylation, thereby reducing the possibility of methyl ester formation at the acid function compared to previous processes. Further, the addition of the hydrophobic benzyl group prior to methylation assists in intermediate isolation, as extraction in the presence of aqueous phases is better than without the benzyl group. De-protection and acetylation of IV then yields the required lacosamide in good overall yield and chiral purity (Scheme 6).

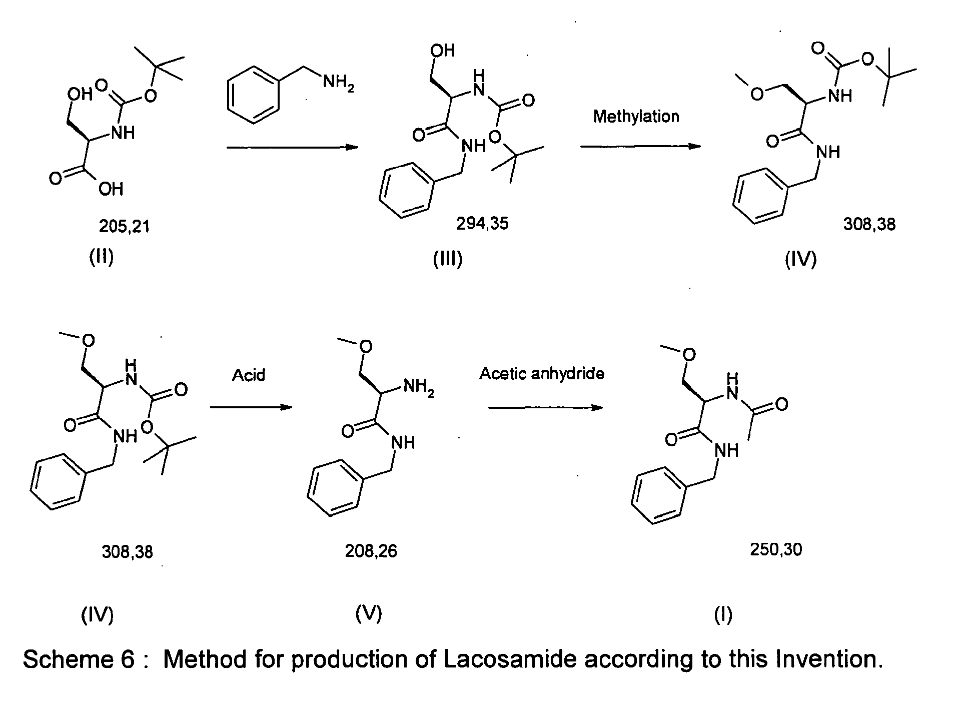

In US5773475 (and WO 2006/037574) it is shown that benzylamine coupling to acetyl-D-serine, prior to methylation, using isobutyl chloroformate at -78 °C results in formation of various impurities that must be removed by chromatography. This leads to a complex process with low yield. This is expensive to operate at scale. It has been surprisingly found that when using the boc- protecting group and using suitable coupling procedures, coupling of boc-D-serine (II) with benzylamine can be readily carried out, and highly pure intermediate isolated by crystallisation. Use of this material in the subsequent methylation reaction leads to good conversion, with minimal racemisation and minimal by-product formation.

Preparation of (R)-boc-2-amino-N-benzyl-3-hydroxy-propionamide (III) from boc-(D)-serine (II) may preferably be carried out using the coupling reagent cyclic propane phosphonic acid anhydride (T3P^{®}, 2,4,6-tripropyl-2,4,6-trioxo-1,3,5,2,4,6-trioxatriphosphorinane) under standard coupling conditions. Thus, the boc-(D)-serine is dissolved in a suitable solvent. Suitable solvents include, but are not limited to, dichloromethane or an ester such as ethyl acetate. To the reaction mixture is added a base, at typically between 2 and 5 mol equivalents. Common bases for such reactions are triethylamine, diisopropylethylamine, N-methylmorpholine or 2,6-lutidine. N-methylmorpholine has been found to work well, however other bases may readily be tested for use. Benzylamine is added at preferably 1-1.5 mol equivalents, though larger amounts may also be added. Indeed, as benzylamine is inexpensive, the use of benzylamine as both a base and source of amine for the coupling is possible. The reaction is typically run under cold conditions (2-8 °C), however alternative temperatures are possible. T3P^{®} is then added to the reaction. At completion of conversion, the reaction is quenched, typically by the addition of water. The product may then be isolated using standard extraction procedures.

For higher purity product crystallisation is preferred. Suitable solvents for crystallisation include, but are not limited to, toluene or an ester solvent mixed with an ether. Alternatively the formed (R)-boc-2-amino-N-benzyl-3-hydroxy-propionamide (III) may be used directly without solid isolation in the methylation step, thereby reducing solids handling. Thus if dichloromethane (or other suitable solvent) is used as a solvent in the coupling reaction, the product (III) is already in a solvent suitable for the methylation reaction.

Although T3P^{®} is the preferred reagent to perform this coupling reaction, other amide coupling reagents like isobutylchloroformate and dicyclohexylcarbodiimide may also be used.

Methylation of (R)-boc-2-amino-N-benzyl-3-hydroxy-propionamide (III) has been found to work well using standard conditions to those described for methylation of boc-(D)-serine using dimethyl sulfate. However, this should not be considered limiting and other methylating reagents (like methyliodide or methyltosylate) and conditions may also be developed within the scope of this invention.

A further aspect of the invention is the conversion of (R)-boc-2-amino-N-benzyl-3-methoxy-propionamide (IV) to lacosamide in a single reactor without isolation or extraction of intermediate (V). W02006/037574 describes removal of the boc protecting group using concentrated hydrochloric acid, followed by extraction of (R)-2-amino-N-benzyl-3-methoxypropionamide (V). Due to its relatively good solubility in water, isolation of (V) requires a phase separation and 2 subsequent extractions of the aqueous phase with dichloromethane (methylene chloride) in order to obtain a good yield. EP2067765 also describes the removal of the protecting trityl group by reaction under acidic conditions. In the procedures described, 2 extractions are also required for good product isolation. In both applications, the isolated (R)-2-amino-N-benzyl-3-methoxypropionamide is then acetylated with acetic anhydride. Surprisingly it is found that acetylation of (V) may be carried out efficiently under aqueous conditions. Thus at the completion of the de-protection reaction with concentrated HCI, no separation of phases, or extraction of product V ((R)-2-amino-N-benzyl-3-methoxypropionamide) is required prior to acetylation. At completion of the de-protection, the pH of the reaction is made alkaline by addition of a base such as sodium hydroxide (or other metal hydroxide) or triethylamine (or other tertiary amine base) and the acetylation reaction carried out directly by acetic anhydride addition, without any separation of phases. Despite the lack of an isolation step the reaction goes cleanly with minimal formation of impurities. Since it has relatively poor water solubility, isolation of the lacosamide is then straightforward by extraction into a suitable solvent such as dichloromethane, washing, solvent exchange and crystallisation. Suitable solvents for crystallisation are already well described and include ethyl acetate and other ester solvents, ethyl acetate/alkane mixtures, toluene or toluene/alcohol mixtures or ether solvent/alcohol mixtures. Such a single reactor process for carrying out the 2 steps of de-protection and acetylation is clearly advantageous over the prior art in reducing reaction time, avoiding the need for extensive extraction of (R)-2-amino-N-benzyl-3-methoxypropionamide (V) and gives potential yield improvements.

### Examples

### 1. (R)-boc-2-amino-N-benzyl-3-hydroxy-propionamide (III)

In a 4 L flask with overhead stirrer, thermometer, dropping funnel and under nitrogen were combined 150 g boc-D-serine (0.731 mol), 1.5 kg ethyl acetate and 221.5 g 4-methylmorpholine (2.19 mol). The reactor was cooled in an ice bath to 4-8 °C and 129.4 g benzylamine (1.24 mol) slowly added. After complete addition a thick slurry is obtained. 525 g of T3P^{®} in ethyl acetate (2,4,6-tri-n-propyl-2,4,6-trioxo-1,3,5,2,4,6-trioxatriphosphorinane as a 50% w/w solution in ethyl acetate, 0.826 mol) were slowly added, maintaining the temperature below 15 °C. The mixture was cooled and stirred on ice at 4-8 °C for 1.5 hours, then at 25 °C for 2 hours. The reaction was quenched by the addition of 400 ml water and stirred for a further 30 minutes. The phases were separated and the aqueous phase re-extracted with 750 ml ethyl acetate. The combined organic phases were washed 200 ml water, adjusted to pH 6 with 37% HCl, then washed a second time with 150 ml water. The ethyl acetate was stripped and exchanged to toluene. The product was crystallised from 1 kg toluene by cooling to 4 - 8 °C. The product was filtered and washed with 2 x 100 ml toluene. After drying under vacuum at 40 °C, 153.8 g (yield 71.5%) (R)-boc-2-amino-N-benzyl-3-hydroxy-propionamide (III) were obtained. HPLC purity >99% , ee 99%, ¹H NMR (400 MHz, CDCl₃): δ = 1.42 (s, 9 H), 3.12 (br, 1 H), 3.65-3.69 (m, 1 H), 4.12-4.18 (m, 2 H), 4.36-4.53 (m, 2 H), 5.62 (d, J = 6.0, 1H), 7.08 (br, 1 H), 7.21-7.36 (m, 5 H).

### 2. (R)-boc-2-amino-N-benzyl-3-methoxy-propionamide (IV)

In a 4 L flask with overhead stirrer, thermometer, dropping funnel and under nitrogen were combined 120 g (R)-boc-2-amino-N-benzyl-3-hydroxy-propionamide (III) (0.407 mol) and 1 kg dichloromethane. The reaction was cooled on ice to 4-8°C and 6.9 g tetrabutylammonium bisulfate (6.8 mmol) and 285 g of 20% sodium hydroxide in water (0.475 mol) added. To the reaction was then added 154 g (1.22 mol) dimethyl sulfate and the reaction stirred at 4-8 °C for 4 hours and overnight at 20-25 °C. A sample showed greater than 98% methylation of the starting (R)-boc-2-amino-N-benzyl-3-hydroxy-propionamide. The reaction was quenched by additing 330 g of 25% ammonium hydroxide solution in water plus a further 330 g water and stirred for 2 hours. The phases were then separated and the aqueous phase extracted with 340 g dichloromethane. The combined dichloromethane phases were washed with 450 ml water, then the dichloromethane was exchanged for tert-butyl methyl ether

(MTBE) and the product crystallised from 345 ml MTBE at 4 °C overnight. The crystals were filtered and washed 2 times with 45 ml MTBE and dried to yield 95.3 g (75.8% yield), HPLC purity = 98.6%, ee >99% ,¹H NMR conforms.

### 3. Lacosamide ((R)-2-acetamido-N-benzyl-3-methoxypropionamide (I))

In a 1 L flask with overhead stirrer, condenser, thermometer and dropping funnel were combined 40 g (0.13 mol) (R)-boc-2-amino-N-benzyl-3-methoxy-propionamide (IV) and 400 ml dichloromethane. To this was carefully added 51.3g (0.52 mol) 37% w/w hydrochloric acid in water. The reaction was stirred at 25 °C for 3 hours, at which time a sample showed >99% hydrolysis of the boc protecting group. To the reaction was added 112 g water. The solution was then cooled on ice to 4-6 °C. With good mixing 75 g (0.62 mol) 33% w/w sodium hydroxide solution in water was then slowly added maintaining the temperature between 4-8 °C. At completion of addition, 17.3 g (0.17 mol) acetic acid anhydride was slowly added. The reaction was allowed to come to room temperature and stirred for a further 1 hour. The phases were separated and the aqueous phase extracted with 100 ml dichloromethane. The dichloromethane phases were combined and washed with 25 ml water. The dichloromethane was then exchanged for toluene and lacosamide crystallised from a mixture of 460 ml toluene plus 10 ml methanol by warming until all solids were in solution and then slowly cooling to 4-8 °C. The lacosamide was filtered and washed 2 times with 50 ml toluene. Yield 24.8 g (76.4%), purity by HPLC 99.9%, ee >99.9%, ¹H NMR conforms.

ee is enantiomeric excess and is calculated by determining the percentage of each separate enantiomer of a given chiral compound, such that the sum of the (R) and (S) enantiomers is 100%, and subtracting one from the other.

## Claims

1. (R)-1-Benzylcarbamoyl-2-hydroxy-ethyl)-carbamic acid tert-butyl ester (compound III) with an ee of greater than 90%.

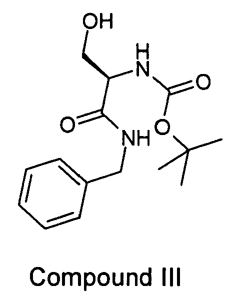

2. ((R)-1-Benzylcarbamoyl-2-hydroxy-ethyl)-carbamic acid tert-butyl ester (compound III) with an ee of greater than 97%.

3. A process for producing (R)-boc-2-amino-N-benzyl-3-methoxy-propionamide in which Compound III, defined in Claim 1, is methylated to produce (R)-boc-2-amino-N-benzyl-3-methoxy-propionamide with an enantiomeric excess greater than 90% with respect to the (R) enantiomer.

4. The process according to Claim 3 in which the ee of the (R)-boc-2-amino-N-benzyl-3-methoxy-propionamide is greater than 97% with respect to the (R) enantiomer.

5. A process for the production of lacosamide in which (R)-boc-2-amino-N-benzyl-3-methoxy-propionamide produced according to Claim 3 or 4 is treated to remove the tert-butoxy-carbonyl- protecting group and then acetylated.

6. A process for the production of lacosamide according to Claim 5 in which (R)-boc-2-amino-N-benzyl-3-methoxy-propionamide is converted to lacosamide without separation of an organic phase containing the intermediate, (R)-2-amino-N-benzyl-3-methoxypropionamide prior to acetylation.

7. A process according to either Claim 5 or 6 in which the lacosamide formed has an ee greater than 90%.

8. A process according to either Claim 5 or 6 in which the lacosamide formed has an ee greater than 97%.

9. A process according to either Claim 5 or 6 in which the lacosamide formed has an ee greater than 99%.
